(19) **Europäisches Patentamt / European Patent Office / Office européen des brevets**

(11) **EP 2 136 697 B1**

(12) **EUROPEAN PATENT SPECIFICATION**

(45) Date of publication and mention
of the grant of the patent:
**26.12.2012   Bulletin 2012/52**

(21) Application number: **08743781.0**

(22) Date of filing: **12.03.2008**

(51) Int Cl.:
*A61B 18/22* (2006.01)          *A61B 1/06* (2006.01)
*A61B 1/07* (2006.01)           *A61N 5/06* (2006.01)
*G02B 23/24* (2006.01)          *G02B 26/10* (2006.01)
*H04N 9/10* (2006.01)           *G02B 26/08* (2006.01)

(86) International application number:
**PCT/US2008/056589**

(87) International publication number:
**WO 2008/112723 (18.09.2008 Gazette 2008/38)**

(54) **MEDICAL DEVICE INCLUDING SCANNED BEAM UNIT FOR IMAGING AND THERAPY**

MEDIZINISCHE VORRICHTUNG MIT ABGETASTETER STRAHLEINHEIT FÜR BILDGEBUNG UND THERAPIE

DISPOSITIF MEDICAL COMPRENANT UNE UNITE FAISCEAU DE BALAYAGE POUR L'IMAGERIE ET LE TRAITEMENT

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **12.03.2007   US 716806**

(43) Date of publication of application:
**30.12.2009   Bulletin 2009/53**

(73) Proprietor: **Ethicon Endo-Surgery, Inc.**
**Cincinnati, OH 45242 (US)**

(72) Inventors:
• **WEIR, Michael P.**
  **Blanchester, Ohio 45107 (US)**
• **DUNKI-JACOBS, Robert J.**
  **Mason, Ohio 45040 (US)**
• **TEOTIA, Neeraj P.**
  **Cincinnati, Ohio 45208 (US)**
• **RITCHIE, Paul G.**
  **Loveland, Ohio 45140 (US)**

• **BROPHY, Jere J.**
  **Loveland, Ohio 45140 (US)**
• **CROPPER, Michael S.**
  **Edgewood, Kentucky 41017 (US)**
• **HUITEMA, Thomas W.**
  **Cincinnati, Ohio 45241 (US)**
• **LONG, Gary L.**
  **Cincinnati, Ohio 45227 (US)**
• **TRUSTY, Robert M.**
  **Cincinnati, Ohio 45241 (US)**

(74) Representative: **Tunstall, Christopher Stephen**
**Carpmaels & Ransford**
**One Southampton Row**
**London**
**WC1B 5HA (GB)**

(56) References cited:
**US-A- 5 200 819          US-A1- 2005 020 926**
**US-A1- 2006 195 014**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Technical Field**

The present application relates generally to medical devices and in particular to a medical device including a scanned beam unit configured for imaging and therapy.

**Background**

Various imaging devices have been used in medical procedures to allow a doctor to view a site within a patient. One such device described in U.S. Patent Publication Number 2005/0020926 is a scanned beam imaging system that utilizes a plurality of radiation sources, the outputs of which are sent to a distal tip via one or more optical fibers. The radiation is scanned across a field-of-view (FOV). The radiation reflected, scattered, refracted or otherwise perturbed within the FOV is gathered and converted into separate electrical signals that can be combined either electronically or through software and used to generate a viewable image.

US 2006/0195014 discloses a capsule coupled to a tether that is manipulated to position the capsule and a scanner included within the capsule at a desired location in a patient's body. Images produced by the scanner can be used to detect Barrett's Esophagus (BE) and early (symptomatic) esophageal cancer after the capsule is swallowed and positioned with the tether. The scanner moves in a desired pattern to illuminate a region. Light from the region is then received by detectors in the capsule, or conveyed to external detectors.

US 5 200 819 discloses an image acquisition system which employs detectors in conjunction with an optical scanner that provides a beam of radiation tracing out a raster to illuminate an object. The system generates data indicative of a two- or three-dimensional image of the object. The image system includes a digital system able to provide a data matrix of two- and three-dimensional coordinates and the radiation intensity at each of the coordinates as well as spectral-dependent image processing, display and archival storage.

US 2005/0020926 discloses a scanning endoscope which scans a beam of light across a field-of-view, collects light scattered from the scanned beam, detects the scattered light, and produces an image. The light sources may include laser emitters that combine their outputs into a polychromatic beam.

**Summary**

The invention provides a medical device according to claim 1. The dependent claims set out optional features.

**Detailed Description**

An embodiment of a medical device includes a scanner assembly, a collector, a radiation source assembly, a detector assembly, a controller and a user interface. The radiation source assembly, detector assembly, controller and user interface make up a functional element that is known herein as a "console." The radiation source assembly, as selected by the user via the user interface, and acting through the controller, generates at least two wavelengths of radiation (e.g., in the visible wavelength range and/or otherwise). This radiation is conveyed in a beam to the scanner assembly, which causes the beam to be swept across a tissue surface. The extent of this swept area is generally known as the "field of view" (FOV). Radiation reflected from the scene within the FOV may be intercepted by the collector and passed to the detector assembly. The detector assembly converts the received radiation to electrical signals that are then configured by the controller to form an image on a display device in the user interface.

The source assembly includes multiple sources, each capable of generating radiation at a selected wavelength. Five sources may be included. The outputs of the radiation sources may, in some embodiments, be brought together in a combiner element to yield an output beam. The combiner may also include beam-shaping optics such as one or more collimating lenses and/or apertures. The sources may be of various types such as, for instance, light emitting diodes (LEDs), lasers, thermal sources, arc sources, fluorescent sources, gas discharge sources, or others. Signals may be provided by the controller to one or more of the sources and optionally the combiner. The signals may optionally control wavelength, power, modulation or other beam properties. The wavelength of radiation, for example, may be selected for imaging, therapy, or aiming. As used herein, an "imaging beam" refers to radiation selected for use in creating an image of a surface or region, a "therapeutic beam" refers to radiation selected to provide treatment of a condition such as diseased or damaged tissue, and an "aiming beam" refers to radiation selected to accentuate a portion of the FOV. In this example, first, second and third sources emit red, green and blue radiation; a fourth source emits an aiming beam at a wavelength selected to yield a distinct contrast to the typical target material; and a fifth source emits a therapeutic beam at a wavelength that is highly absorbed and moreover can be efficiently generated at high power to treat diseased or damaged tissue. In some embodiments, the aiming beam may be provided by source separate from the therapeutic beam source. As an alternative, an aiming beam may be provided by the fifth source as a reduced power therapeutic beam. In some embodiments, the aiming beam could be a virtual beam (i.e., a region in which one or more of the imaging sources is caused to increase (or decrease) significantly to create a bright (or dark) region in the displayed image.

In some embodiments, a source provides a diagnostic beam. A "diagnostic beam" as used herein refers to radiation selected for analysis or detection of a disease or other medical condition including, for example, to visualize the presence of (or to activate) a diagnostic marker. The diagnostic marker could be naturally occurring (e.g., auto or self fluorescence) or introduced as part of the diagnostic procedure (e.g., fluorescent dyes).

Use of an aiming beam may be preferred in some circumstances. As will be seen later, while the treatment beam may follow the same path as the imaging beam, it is not constrained to follow the same timing. An aiming beam, managed in the same way as the therapeutic beam though at lower power and in a visible wavelength, may help ensure that the treatment is applied where the user intends. Furthermore, it may be a requirement of certain industry or regulatory standards such as AAMI or IEC that where higher power lasers are employed, an aiming beam be provided.

It should be noted that while five sources may be included, there may be more or fewer emitters depending, for example, on the end use. In some embodiments, sources may be combined or capable of providing various types of energy. In some cases, filters may be used to filter the radiation. In some embodiments, the first, second and third sources comprise three lasers; a red diode laser, a green diode-pumped solid state (DPSS) laser, and a blue DPSS laser at approximately 635 nm, 532 nm, and 473 nm, respectively. While laser diodes may be directly modulated, DPSS lasers generally require external modulation such as an acousto-optic modulator (AOM) for instance. In the case where an external modulator is used, it is considered part of the radiation source assembly and not shown separately.

The operation of a device incorporating a scanner assembly will now be described. A reflector, part of the scanner assembly to be described in more detail later, receives a beam of radiation from source assembly and directs the beam onto the surface, for example, for one or more of imaging, therapy, or aiming purposes. At one point in time, the beam deflected by the reflector impinges upon the surface to illuminate a first point. The reflector oscillates in at least one axis (two axes in some embodiments) so that at some other point in time the deflected beam illuminates a second point. Radiation is, in general, reflected, absorbed, scattered, refracted or otherwise affected by the properties of the surface. Radiation may leave the surface in many directions. The collector, however, may only capture that fraction of radiation which falls into the area subtended by its aperture.

The detector assembly will now be described. Radiation that is intercepted by the collector is passed to the detector assembly. This radiation includes energy at several wavelengths, corresponding to those emitted by the source assembly, and possibly also including other wavelengths as may result from nonlinear processes (such as fluorescence). In some embodiments, wavelength separator separates the incoming radiation into pathways. Such separation may be performed by filters, gratings, or other devices. In an alternate configuration, wavelength separation may be incorporated in the collector, and separated wavelengths brought to the detectors, each in its own fiber or fiber bundle. Each separated wavelength of radiation is then sent to the detectors in the detector assembly. Such detectors may be physically separate, or parts of a common detector such as a CCD or CMOS device. Multiple detectors may be incorporated for each wavelength. The detectors output electrical signals corresponding to the power, amplitude, or other characteristic of each wavelength of radiation detected. The signals can be used by the controller to generate a digital image, e.g., for processing, decoding, archiving, printing, display, etc.

In some embodiments, an input X to the detectors is capable of modifying the transfer function from radiation to electric signals. Exemplary modifications may include adjustment of gain or offset or both. An input Y to the wavelength separator may be capable of modifying the transfer function therethrough. The modifying elements X and Y may be disposed to operate on the input to the respective detectors and the wavelength separator, acting on all or a subset of wavelengths received, at the outputs of the respective detectors and the wavelength separator or at both inputs and outputs.

The controller will now be described. An interface management component, among other tasks, accepts operating mode commands from the user. Such commands may include imaging and treatment modes, FOV and/or aspect ratio of the image, image storage, etc. Specifications related to the FOV and aspect ratio result in parameters sent to a scanner driver, which generates requisite drive signals to the reflector. The user may also specify treatment parameters, such as the location, shape and size of a region to be treated, the wavelength to be used, and duration of exposure. These result in parameters being sent to a coordinate converter, which converts the specifications into selection and modulation commands to a source control and modulation block. This source control and modulation block drives the source assembly to provide the requisite radiation outputs. Signals from the detector assembly are converted from their scan coordinate system to a Cartesian form for display and sent to the interface management block for user viewing. Details of this conversion procedure are described later.

In some embodiments, motion sensing is incorporated within the system. For example, a first element may include a number of sensors attached or connected to the scanner assembly. The sensors may sense location, orientation or both. The sensors may be, for example, accelerometers, magnetometers, rate gyros, electromagnetic position sensors, etc. Location and orientation signals are generated by the sensors. A mathematic operation is capable of converting the location and orientation signals into a stationary reference frame. Output of the mathematic operation is used to modify the relationship of a displayed image to the scanned data to compensate for sensed movement.

An element operates on the scanned data to detect the relative movement and provides signals indicating magnitude and direction of the movement. This image tracking functionality may provide reliable treatment of the body which might be moving due to, for example, respiration, circulation or other biological activity.

The scanner assembly will now be described. The scanner assembly includes a housing that encloses the reflector and other components. A source fiber is used to deliver energy from the source assembly to the scanner assembly. The source fiber may be a single mode optical fiber. In some embodiments, one or more fibers may be used to deliver imaging beams and one or more other fibers may be used to deliver a therapeutic beam (e.g., therapeutic beams having longer wavelengths, e.g., greater than 1700 nm and/or higher power). In certain embodiments, a different type of fiber, such as a holey fiber, may be used to transmit energy from the source assembly. In some embodiments, the same optical fiber is used to deliver both the imaging beams and the therapeutic beams to the reflector, the optical fiber defining a common path for both types of beams.

Electrical wires convey drive signals for the reflector and other signals (position feedback, temperature, etc.) to and from the scanner driver. The wires may also provide control and feedback connections for controlling focus characteristics of the beam shaping optic. The distal end of the scanner assembly is fitted with an optical element which allows the scanned beam to pass out and illuminate the scene. This element may be a dome; however, its curvature, contour, and surface treatments may depend on the application and optical properties required. In some embodiments, the dome provides a hermetic seal with the housing to protect the internal elements from the environment.

The internal components of an embodiment of the scanner assembly will now be described. The source fiber is affixed to the housing using a ferrule. The end of the source fiber may be polished to create a beam of known divergence. The beam is shaped by a beam shaping optic or lens to create a beam shape appropriate for transmission through the system. After shaping, the shaped beam is fed through an aperture in the center of the reflector, then reflected off a first reflecting surface. The first reflecting surface may have a beam shaping function. The beam is then directed onto the reflector and then out of the scanner assembly, the details of which (in the case of an imaging beam) are described in U.S. Patent Application Serial No. 10/873,540, entitled SCANNING ENDOSCOPE. Any suitable materials can be used to form the reflector. In some embodiments, the reflective surface of the reflector may be formed of gold or other suitable material for directing each of the beams including relative high energy therapeutic radiation. In other embodiments, a multilayer dielectric configuration may be used in forming the reflector.

An embodiment of the collector will now be described, which in this case is configured to be installed coaxially with the scanner assembly. Radiation reflected from a scene impinges on the face of the collector, which constitutes the receiving aperture. The face is actually made up of the polished ends of a large number of small diameter, multimode collecting fibers which conduct the radiation to the detector assembly. The scanner assembly is inserted into a central void. The collector is enclosed by a housing. The fiber ends making up the face may be formed in a plane, or into other geometries to control the pattern of receiving sensitivity. They may be coated with diffusing or other materials to improve their angle of acceptance, to provide wavelength conversion, or wavelength selectivity. In some embodiments, the detector assembly may be configured to form the receiving aperture and mounted in position to receive the reflected radiation directly, without the need for a separate collector.

An exemplary endoscope for medical use will now be described. The endoscope generally includes an elongate, rigid or flexible shaft having a distal end and a proximal end opposite the distal end. There is typically a handle which includes a number of controls, often both mechanical and electrical. The endoscope is connected to the console by source fibers, collection fibers, and the electrical wiring. As used herein, an endoscope refers to an instrument for use in examining, diagnosing and/or treating tissue comprising a patient's body, either percutaneously or through a natural orifice or lumen. As used herein, the term "proximal" refers to a location on the medical device nearer to a user, and the term "distal" refers to a location that is nearer the patient. Typically, the console of the medical device is located outside a patient's body and the distal end of the medical device is insertable into the patient's body. However, other configurations are possible. Furthermore, while an endoscope is referred to, any suitable type of medical device may be employed such as gastroscopes, enteroscopes, sigmoidoscopes, colonoscopes, laryngoscopes, rhinolaryoscopes, bronchoscopes, duodenoscopes, choledochoscopes, nephroscopes, cystoscopes, hysteroscopes, laparoscopes, arthroscopes, etc.

An embodiment of a user interface for use in selecting a desired treatment region, will now be described. In one exemplary mode of operation of the system in performing therapy, an image of the scene is displayed on a geometry display device. The controller generates a cursor illustrating where the treatment beam will be emitted. The aiming beam may be enabled to confirm the point of treatment before enabling the treatment beam. In this mode, the treatment beam occupies a fixed position in the display space, and the operator manipulates the scope so as to bring the tissue to be treated into alignment with that beam. The treatment zone is represented as being small, such as might be the case when an incision or cauterization is planned along a line.

In a similar mode of operation to that described above, the user employs a geometry input device to specify a region over which treatment is to take place. The region may be a circle.

In another similar mode of operation, the cursor can be positioned at a location selected by the user. In this embodiment, the device (e.g., the endoscope) is positioned such that the desired treatment point and important other details of the scene are visible in the image. The user can then position the cursor at the location of the desired treatment point (e.g., by touching the geometry display device at the desired location or by using the geometry input device.

In yet another similar mode of operation to that described immediately above, the user employs the geometry input device to specify a region over which treatment is to take place, which may be a circle.

In a further similar mode of operation to that described immediately above, the user specifies an irregular region' of treatment. Such regions may be defined by specification of a number of points, using the geometry input device, between which the system constructs lines or curves defining the treatment boundary, or by stretching and modifying a small number of predefined geometric shapes. In such a mode, the aiming beam is particularly useful in confirming that the treatment region will be where the user intended.

It should be noted that while a single treatment zone or region may be specified, in some embodiments, multiple treatment zones may be specified simultaneously.

In general terms, the user interface will now be described. The expression "value" in this description refers to quantities which can be expressed as simple numbers, text strings or scalar quantities. The expression "geometry" refers to quantities that have a multidimensional or multiparameter nature, such as an image, a vector, an area, or a contour. Commands from an interface management block are displayed for user viewing on the value or geometry display devices. The interface management block may be software (and possibly dedicated hardware) to manage and control the data to and from the devices in the user interface. The interface management block includes control logic to manage the setting of treatment point and, once a treatment is determined and requested, to control the creation of the control sequences to cause treatment through the coordinate converter and the source control and modulation block. Examples of value quantities that might be displayed include operating mode, power output, equipment status, field of view, available image storage space, date, and time. Geometry display quantities include the image of the scene being viewed, treatment regions, boundaries, or paths. Input values include operating mode selection, names of stored image files, and image (color balance, brightness, contrast, enhancement) adjustments. Geometry input quantities include a region or pathway to be treated by a therapeutic beam, or zones in which special image processing is to be performed.

All these functions may be provided in a single multifunction device, such as a touch screen panel, which can accept user input as well as display data of geometric and value types. It may be preferable, however, to provide specialized devices which provide a more ergonomic or haptic match to the operating tasks. For example, a text display might be utilized for the value display, reserving a larger and more expensive graphical display for the geometry display to avoid cluttering images with interfering data. Similarly, while simple pushbuttons or keyboards (virtual or real) may serve to enter both values and geometry quantities, they may be ill suited to the latter. A joystick, trackball or multi-axis device such as used on the Da Vinci surgical robot, available from Intuitive Surgical, Inc. may be used for specifying geometry inputs.

In addition to marking a region and then providing a signal to the scanner assembly to apply the therapeutic beam to that region, a more interactive and immediate treatment mode may be provided, where the geometric input device is used to enable real-time, live application of treatment radiation, typically in a small spot beam such as would be familiar to users of electrocautery and laser cutting devices. Such a mode may be useful in a variety of surgical procedures such as prostate surgery which can be performed under direct visualization without additional cystoscopes, bladder surgery where bladder tumors or bladder cancer can be imaged and thermally necrosed, removal of varicose veins where the endoscope can be inserted into the saphenous vein and used to treat the inside of the vein under direct visualization, destruction of kidney stones where the stone can be directly visualized and then thermally destroyed using a single device, etc.

In one embodiment, a treatment region may be automatically recognized, for example, using the presence of fluorescence or other marker. A signal may then be provided to the scanner assembly to apply the therapeutic beam to that automatically selected treatment region. A disease or tissue specific agent bound to a fluorescent material may be placed into the patient via, for example, the circulatory system that gathers at the target diseased or tissue area. The system can automatically identify a region to be treated by observing, for example, a spectral signature of the fluorescent material. The user may then confirm the treatment region and then authorize treatment to proceed (possibly specifying a treatment dose).

As mentioned above, the reflector scans the beam of radiation in a pattern. High-speed MEMS reflectors and other resonant deflectors as described herein are configured and driven to execute sinusoidal angular deflections in two orthogonal axes, yielding the classical Lissajous pattern. Most current display devices are configured to address display data in a Cartesian form, for example as row and column, or a particular pixel along a nearly-horizontal scan line. The bi-resonant or Lissajous scan path is overlaid with the Cartesian or rectilinear grid. In this instance, the intersections between the vertical and horizontal lines of the Cartesian grid represent display pixel positions while the Lissajous trace represents the actual path taken by the scanned spot. As the actual scan path does not align perfectly with all the rectilinear pixel positions, these image values may be determined through interpolation. In some embodiments, registration of the Lissajous trace to the Cartesian grid is based on a marker that links a reference point in the scan to a point in the rectilinear matrix.

The interaction between a user and the system will now be described. The user defines a treatment zone, border, or path by means of specification while viewing the image. Such specification includes the identification of places in the image, and thus on the target tissue, and selection of parameters such as the treatment beam wavelength, power, and duration of exposure. The specification and the image may be represented as data quantities passed between system elements; they may instead be represented as planar figures.

The user may define a treatment zone, border and/or path to perform and one or more of a variety of medical procedures. A general discussion of various laser treatment modalities using the fifth source follows. This discussion is not meant to be exhaustive and should not be construed as limiting. Generally, laser therapy can be categorized into four areas: (i) Photodynamic Therapy (PDT), (ii) dermal treatment, (iii) thermal ablation and (iv) opto-thermal shock waves. A discussion of complexities involved in designating a treatment zone and delivering the desired treatment to that treatment zone using the scanner assembly follows.

In PDT, a chemical (e.g., porfimer sodium) that preferentially collects at a target organ or tissue type is introduced into a patient, typically intravenously. The chemistry may be such that it is relatively inert until it is activated photonically. A therapeutic laser beam of the appropriate wavelength and power (typically visible wavelengths such as between about 400 nm and 700nm and moderate power such as between about 1 mW and 100 mW) is caused to illuminate the target tissue, which activates the chemical and treats the tissue, typically through oxidative destruction of tumors located in the tissue.

In dermal treatments, a wavelength is typically selected to be preferentially absorbed by the targeted tissue or material to be treated, and energy density is selected to ablate the target material without unduly destroying adjacent tissue. For example, in tattoo removal, different color dyes absorb specific laser wavelengths and the laser power is chosen to vaporize the dye encapsulated in the tissue, causing the dye color intensity to diminish. Tattoo removal using a scanned beam imager is described in U.S. Serial No. 11/615,140, entitled APPARATUS AND METHOD FOR MEDICALLY TREATING A TATTOO, tiled December 22, 2006.

In thermal ablation, specific tissue is targeted for volumetric necrosis. Tissue necrosis is accomplished by subjecting tissue cells to a particular temperature for a particular period of time. Thermal ablation can be sub-categorized into several regimes such as coagulation and vaporization. During heating, the tissue is heated to temperatures generally less than about 41 °C with no lasting effect results. During coagulation, the tissue is heated to between about 41 °C and 100 °C, and cell death occurs based on the amount of time the tissue is subjected to the temperature. Generally in coagulation, a wavelength may be chosen to maximize tissue penetration depth to evenly heat a volume, for example, in the near infrared between about 700 nm and 1050 nm and at lower power levels, such as between about 1 W and 50 W. In vaporization, a wavelength is typically chosen to be absorbed at the surface of the targeted tissue, and the low volume of cells at the tissue surface experience rapid temperature rise above 100 °C, and the tissue is immediately denatured and vaporized. For vaporization, power levels can vary greatly based on the energy density delivered to the tissue, but are typically between about 1 W and 50 W. In opto-thermal shock, a laser is chosen with a fast pulse time such that very high instantaneous energies are used to create cavitation bubbles that collapse quickly and send a mechanical shock wave through targeted tissue. This type of treatment is typically used in laser lithotripsy to break up stones or calcification sites in the patient. Q-switched Nd:YAG (e.g., 1060 nm) or Alexandrite (e.g., 380 nm, 760 nm, 1440 nm), erbium:YAG (or Ho:YAG, e.g., 2112 nm) lasers may be suitable for opto-thermal shock treatment with sub-microsecond pulse times (e.g., 8 ns). Flash-lamp-pulsed dye lasers may also be suitable with longer pulse times on the order of 1-250 us. In some cases, CW lasers may be used in lithotripsy to heat a stone directly to cause stress-induced breakage.

Therapeutic beam modulation may be employed to deliver the desired amount of therapeutic radiation as the reflector moves along its scan path. Generally, a beam which has been deflected by a mechanically-resonant reflector moves through space at a varying velocity. When this beam impinges upon a target, the time spent in any one area may differ across the FOV. Additionally, the size of the spot (or footprint) on the target may vary with the target's distance and inclination to the beam, which can cause the flux to vary.

As can be appreciated, complexities may arise when both imaging and delivering therapeutic radiation. While collecting image data, the illumination power may be on the order of milliwatts or tens of milliwatts, depending on the application requirements (working range, field of view, etc.). The treatment power, on the other hand may be in the range of watts or tens of watts. The treatment power may be delivered at wavelengths outside the visible range, or within the visible range, and may even be within the range of those wavelengths used for imaging. It will be apparent that even though the treatment wavelengths are selected for tissue effect, meaning they must be significantly absorbed into the tissue, the target may reflect significantly higher treatment energy than imaging energy.

All systems having inputs, but particularly receiving and detecting systems, may be characterized by their dynamic range. Various definitions are used depending on context and application, but all include the notion of a range of signal levels over which they operate correctly. Typically, if signals are below the lower limit of the range, they are not seen as distinguishable from noise. If the signals are above the upper limit of the range, then they will not be correctly recognized. In many detection systems, such as would be employed in a scanned beam system, the detection system may be "saturated" or "paralyzed" by signals above the upper limit, meaning that the detection system does not respond even to signals within its dynamic range, for some extended period of time. The detection system may recover to full functionality after some prolonged period of recovery. If signals are too high, the detection system may be permanently damaged. "Overload" is a term often applied to situations where the signal is beyond the upper limit of the dynamic range, and the overload may be so large as to damage the detection system.

When treatment wavelengths are well separated from those employed for imaging, high-pass, low-pass, and band-pass filters may be appropriately used to inhibit any damaging amount reflected treatment power making its way through the receiving elements to the imaging detectors. When the treatment wavelengths are near the imaging wavelengths, however, filters may be of less utility because of practical constraints on the accuracy, sharpness and stability of their transfer characteristics. Furthermore, even when the wavelengths are well separated, the amount of attenuation out of band is not infinite, and some treatment energy may leak into the imaging system. Finally, since the treatment and imaging beams are likely to be in close proximity, sharing deflection and other system components, the probability of scattering some treatment energy into the imaging system even before it impinges on the target is high.

Thus, it may be advantageous to design for some small amount of the treatment energy to leak into the imaging system, for it provides irrefutable confirmation of the region experiencing treatment. Nevertheless, it may be appropriate to employ further measures to inhibit excessive disruption of the receiving system. In many cases, it may be the detector elements which are most susceptible. A number of means for inhibiting disruption of the detection system may be employed. For example, the sensitivity of the detectors may be reduced when the treatment energy is applied, for example by reducing or removing bias on avalanche photodiodes. The amount of energy input to the detectors may be attenuated, for example by using electrooptic modulators. The input to the detectors may be completely blocked, for example, by using MEMS devices such found in the Texas Instruments Digital Light Projector to deflect the energy away from the detectors.

In addition to protecting the detector from overload, circuitry following the detector may also be configured to prevent generation or propagation of any large transient that accompanies the systems and processes just described. A suitable approach, for example, is to preserve and hold the signal present just before the onset of treatment, and return to following the input when the treatment ceases. This mechanism may be applied immediately after the detector in the signal processing chain. Further modifications of the signal, for example, to show a pseudocolor or other indicator of treatment in progress, may be applied here as well, or later in the signal processing chain.

Some embodiments use a micro-electromechanical (MEMS) scanner reflector to direct the imaging, aiming and therapeutic beams onto the surface. MEMS scanner reflectors are described in, for example, U.S. Patent No. 6,140,979, entitled SCANNED DISPLAY WITH PINCH, TIMING, AND DISTORTION CORRECTION; U.S. Patent No. 6,245,590, entitled FREQUENCY TUNABLE RESONANT SCANNER AND METHOD OF MAKING; U.S. Patent No. 6,285,489, entitled FREQUENCY TUNABLE RESONANT SCANNER WITH AUXILIARY ARMS; U.S. Patent No. 6,331,909, entitled FREQUENCY TUNABLE RESONANT SCANNER; U.S. Patent No. 6,362,912, entitled SCANNED IMAGING APPARATUS WITH SWITCHED FEEDS; U.S. Patent No. 6,384,406, entitled ACTIVE TUNING OF A TORSIONAL RESONANT STRUCTURE; U.S. Patent No. 6,433,907, entitled SCANNED DISPLAY WITH PLURALITY OF SCANNING ASSEMBLIES; U.S. Patent No. 6,512,622, entitled ACTIVE TUNING OF A TORSIONAL RESONANT STRUCTURE; U.S. Patent No. 6,515,278, entitled FREQUENCY TUNABLE RESONANT SCANNER AND METHOD OF MAKING; U.S. Patent No. 6,515,781, entitled SCANNED IMAGING APPARATUS WITH SWITCHED FEEDS; U.S. Patent No. 6,525,310, entitled FREQUENCY TUNABLE RESONANT SCANNER; and U.S. Patent Application Serial No. 10/873,540, entitled SCANNING ENDOSCOPE.

The user defines a treatment zone, border, or path by means of the specification while viewing the image. Such specification may include the identification of places in the image, and thus on the target tissue. This action can be performed through tracing paths on the geometry display device, utilizing the geometry input device as noted above. These paths generally follow the periphery of regions to be treated. The controller can maintain and mark the selected path, and allow adjustment and editing of the path.

A further task in establishing the treatment domain is selection of parameters such as the treatment beam wavelength, power, and duration of exposure. In some embodiments, the operator utilizes the value input device to complete these tasks.

The following discussion describes how specification of points in the display space, from which lines and then areas may be specified, may be mapped to the acquisition space. The discussion begins with mapping from scan coordinates to display coordinates and then from display coordinates (e.g., where a user has specified a treatment region) to scan coordinates (e.g., where the treatment radiation is to be applied).

Scan Coordinate to Display Coordinate Mapping

The scanner assembly employs the oscillating reflector with two orthogonal axes of rotation (x and y) that operate in a resonant mode. The rate of oscillation is typically higher in one axis than the other. When properly excited, the oscillating reflector causes a beam of light reflected from its surface to trace a Lissajous pattern. The coordinates of the beam are approximated by

$$x(t) = A \sin(w_f t + \phi_f)$$

$$y(t) = B \cos(w_s t + \phi_s).$$

Based on the phase relationship of the slow and fast axis motion, the basic Lissajous pattern can precess. The number of slow axis cycles required to precess the pattern to an initial spatial point, is called the interleave factor.
The Lissajous pattern is spatially repeated after a set number of oscillations on the slow axis (interleave factor). Once a reference point on the complete set of Lissajous patterns is identified, one can view the constant sample time, digital data stream captured at each optical detector as a vector of constant length, the Scanned Data Vector ($SDV_i$). The number of samples in the vector (N) is equal to the interleave factor times the period of the slow axis oscillation divided by the sample interval ($t_s$).

$$SDV_i(j \Delta t) = [s(i,j)]_{j=0}^{N-1}$$

If there are multiple optical detectors sampled coincidently, then the scanner assembly data stream can be viewed as a matrix, the Scanned Data Matrix (*SDM*), that has a row count equal to the number of sampled detectors (*M*) and a column count equal to the number of samples in each SDV (*N*). In a system having three color plus fluorescence channels,

$$SDM = \begin{bmatrix} SDV_R \\ SDV_G \\ SDV_B \\ SDV_F \end{bmatrix}.$$

The pixel data matrix (*PDM*) is a two-dimensional matrix with row and column indices that represent the display space. In the above-described scanner assembly, for example, there may be 600 rows (Y) and 800 columns (X) and each point in the data set may be a triple representing red (R), green (G), and blue (B) display intensities.

$$PDM = \begin{bmatrix} (r_{0,0}, g_{0,0}, b_{0,0}) & \cdots & (r_{0,799}, g_{0,799}, b_{0,799}) \\ & \ddots & \\ & & \\ (r_{599,0}, g_{599,0}, b_{599,0}) & & (r_{799,599}, g_{799,599}, b_{799,599}) \end{bmatrix}$$

In order to conveniently describe matrix operations, it may be useful to define a view of the matrix, *PDM,* that is a vector of length XY called *PDV.* The transformation between the two is not a matrix operation, but rather a reordering where the rows of *PDM* are constructed of successive blocks of *PDV.* Note that it is essential that the same reordering be used when accessing the **PDV** and the transformation matrix, **T** to be described next.

One exemplary method for transforming between Lissajous and Cartesian spaces involves multiplication by a matrix **T** or its inverse. The process for constructing this matrix is given in a later section. Matrix **T** is an $N \times XY$ matrix where $N$ is the number of samples in the *SDV X* is the number of horizontal pixels in the display space; and $Y$ is the number of vertical pixels in the display space.

When converting from the Lissajous space **SDM** to the Cartesian space **PDM**, it may be helpful to take a close look at the physical situation from which the data derives.

The beam trajectory may be shown overlaying pixel data. The index into the data samples may be $j$ and pixels may have indices $(k,l)$, corresponding to discrete values of conventional Cartesian coordinates $(x,y)$: not matrix indices (row, column). The origin of the pixel data coordinates may be in the upper left hand corner. Data from a particular data sample will be distributed into pixels falling into a region of radius $r_d$ centered on the sample.

A solid line may represent a portion of a specific trajectory of the dual resonant scanned beam through the scene. The sample index *(j)* may increase from the top left to bottom right in one depiction. The trajectory of the beam (with increasing sample index) can be in any direction through a subset of the scene. The particular sample index on the beam, $m$, will be utilized in subsequent discussions.

Conversion from Lissajous to Cartesian Data space can be represented as a matrix multiplication, followed by a data reordering

$$[SDV][T] = [PDV]$$

where the pixel data vector PDV is then reordered to yield the pixel data matrix PDM. If the number of samples in the **SDV** vector is $N$ and the size of the Cartesian space is $X$ by $Y$, the transformation matrix, **T**, is of dimension $N$ by $(X*Y)$.

The following process can be used to populate the **T** matrix. Through precise knowledge of the path of the scanned beam (that knowledge is assumed to be inherent in the scanner drive and positioning system) it is possible to identify the pixel data point closest to the sample, m, at $t = m\Delta t_s$ from the start of the frame. Denote that pixel with the indices $(k,l)$. Next, construct a circle in Cartesian space of radius, $r_d$ over which the data from sample, $m$, is going to be distributed. For each pixel $(k+s,l+t)$, where $s$ and $t$ are integers that describe points in Cartesian space located within the circle constructed above centered within the circle (a) compute the length (in Cartesian space), $l$, of the vector from the Cartesian space location of the SBI sample, $m$, to the center of the pixel space data pixel, $(k+s,l+t)$ and (b) calculate a weighting value, w, that is proportional to the length, of the vector. Many functions can be used, however, it should be a function decreasing monotonically with distance, such as, for example,:

$$w = e^{-F \cdot \frac{s}{r_d}}$$

where:

w is the weighting factor,
s is the length of the vector from the SBI data point to the pixel of interest
F is a controllable constant that sets how fast the effects of the SBI data falls off as the value of 1 increases.
$r_d$ is the radius of the circle over which the data from the SBI sample is being distributed.

Record the value of w into the transformation matrix T at the x,y location of the subject pixel. The location in the matrix will be at row m and column $j*N + x$. It should be recognized that this method creates a sparse matrix, T. To improve computational efficiency, one may optionally use various methods to create a banded matrix amenable to hardware acceleration or optimized software algorithms, which is described by Hammond S, Dunki-Jacobs R, Hardy R, Topka T. "Architecture and Operation of a Systolic Sparse Matrix Engine", Proceedings of the Third SIAM Conference on Parallel Processing for Scientific Computing, 1987, (419-423).

Display Coordinate to Scan Coordinate Mapping

One can convert from a particular data set, such as an image, in Cartesian space to a sample vector, $m$, by reordering the data into consistent form (that is, a vector of conformable size) and then solving the matrix equation:

$$[\mathbf{SDV}] = [PDV]T^{-1}$$

where T is constructed as shown above. The above equation yields the multi-bit (analog) scan beam vector, **SDV,** which would result from a multi-bit (analog) Cartesian space matrix, **PDM.** Note that, in general, **T** is not square and the creation of the pseudoinverse matrix $T^{-1}$ can be computationally challenging, but can be accomplished as is known in the art. Distribution of multi-bit Cartesian space data to a multi-bit drive (continuously varying modulation) of the scan beam in Lissajous space does require construction of the inverse of the **T** matrix.

For simple ON/OFF control of the scan beam, however, the required mapping can be accomplished by simple inspection of the transformation matrix, **T** as follows. Each column, *j,* of the matrix, **T,** is associated with a specific Cartesian space location, *(x,y),* and contains the weighting function, *w,* for all of the samples in the vector **SBV.** Therefore, the $m^{th}$ row in the column contains the weighting factor, w, for the $m^{th}$, sample in the vector **SBV.** As there might be multiple non-zero cells in the column, the closest sample to a particular location, *(x,y),* will be that row in the column with the largest value, w. By repeatedly performing the above inspection for each pixel (x,y) in the Cartesian space and placing the results at the appropriate location of a mapping matrix, M, that is of dimension Y by X; each cell of M contains the SBV sample number, m, closest to the Cartesian space location, (x,y).

In a simple case, a rectangular treatment region, a subset of the rectangular full FOV of the imaging system, is defined. The pixel data locations *(x,y),* may be denoted by the "+" symbols. The beam track may be denoted by long-dash lines (a-g,A-G). In this case, we wish to turn ON a treatment laser when the scan beam enters a treatment zone (e.g. at $a_0$ or $A_0$) and turn it OFF as the beam exits the treatment zone (e.g. at $a_1$ or $A_1$). It may be noted that the number of samples, and the amount of time that the beam is ON, changes from sweep to sweep.

Event timelines may lead from a representation of the data stream or **SBV** to a synchronized ON/OFF therapy or aiming source control stream. The beam tracks may be in a possible relationship to both the image data steam and treatment control stream. Note that it is not required that the temporal granularity (sampling period) of the data stream and the source control stream be identical. What is required is that the time from the start of the respective streams to the state changes in the source control stream and the target transition times in the data stream are reasonably similar.

In light of the previous discussion of the mapping matrix **M,** it is clear that knowing the pixel locations, *(x,y),* at which the therapeutic or tracer beam enters and leaves the treatment zone can be computed and thereby, the times (from start of the frame) at which the control stream must turn ON and OFF.

There may be limitations on the minimum ON time for the source. Likewise, long ON times could cause system heating or other effects. These limitations might create situations where very short ON times are not honored and where long ON times might be broken into two or more patterns run on sequential frames.

A number of detailed embodiments have been described. Nevertheless, it will be understood that various modifications may be made. For example, in some embodiments, additional imaging beams and/or diagnostic beams are provided. For example, the source assembly may be configured to provide radiation of a pre-selected wavelength, such as blue light (about 370 to about 460 nm), to excite tissue to autofluoresce or to excite an applied chemical to fluoresce. In other embodiments, an imaging beam may not be in the visible wavelength range, for example, a wavelength of about 1600 nm that may allow visualization of tissue structures through layers of other tissue or fluid or may enhance visualization of certain specific tissue types present in a field of blood or other tissue type. A complementary detector may be employed to detect the returned radiation and the controller is configured to display the signals in a chosen color or grayscale.

The scanner assembly may also be used in a variety of skin surface treatments. For example, the scanner assembly may be used for laser hair removal while reducing damage to surrounding skin. A medical device including the scanner assembly may be used to produce an image of the skin surface, identifying a hair shaft, projecting the location and extent of the hair bulb, and the therapeutic laser can be automatically controlled to provide treatment to one or more of the hair shaft, hair follicle, hair bulb and dermal papilla. An acne reduction system can also be provided where the system including scanner assembly is used to eliminate Propionibacterium acnes (P.acnes) bacteria while minimizing damage to surrounding skin. A medical device including the scanner assembly may be used to produce an image of the skin surface, identifying an acne site and the therapeutic laser can be automatically controlled to provide treatment. An acne reduction system can also be provided where the system including the scanner assembly is used to reduce local production of sebum while minimizing damage to surrounding skin. A medical device including the scanner assembly may be used to produce an image of the skin surface, identifying an acne site, projecting the location of the sebaceous gland and the therapeutic laser can be automatically controlled to provide treatment. A skin rejuvenation system can also be provided including the scanner assembly to precisely control laser-based thermal energy to small diameter, high aspect ratio treatment zones with substantial regions of untreated epidermal and dermal skin tissue in a manner that allows rapid, reliable skin rejuvenation, minimizing damage to surrounding skin tissue that can lead to prolonged post procedure recovery. This may be accomplished by verifying density (e.g., treatment zones per $cm^2$) in

an image obtained using a fixed focus scanner system of treatment zones applied to the skin. For portions of tissue that do not contain treatment zones of at least a user prescribed density, therapeutic laser pulses can be generated using the scanner assembly to create additional treatment zones.

In some embodiments, the system may include tracking (e.g., using instrument motion sensors and tissue motion sensors) so that the targetable treatment region or point can move with moving tissue and/or a moving endoscope. In some embodiments, image recognition may be used for target tracking for example by looking for a distinctive feature in the image to act as a reference. In some embodiments, multiple, different control points or regions may be selected within the FOV, for example, to allow for treatment of multiple tissue areas as the reflector moves. Accordingly, other embodiments are within the scope of the following claims.

**Claims**

1.  A medical device comprising:

    a radiation source assembly having at least two radiation sources, where one or more of the radiation sources is adapted to generate an imaging beam for use in visualization of a scene and one or more of the radiation sources is adapted to generate a therapeutic beam for treatment of a medical condition;
    an optical fiber for directing radiation energy from the radiation source assembly toward a distal end of the medical device in the form of a beam;
    a reflector that receives the beam from the optical fiber, the reflector being configured to scan the beam in a pattern, the extent of the pattern formed by the scanned beam defining a field-of-view;
    a receiving system including a detector arranged and configured to receive radiation from the field-of-view to generate a viewable image;
    a controller configured to generate a video image based on electrical signals generated by the detector that correspond to the radiation received by the detector from the field-of-view;
    a geometry input device configured for user specification of a treatment region;
    a display device configured to display the video image of the field-of-view to the user;
    wherein the imaging beam and the therapeutic beam from the at least two radiation sources are directed to follow a common path to the reflector;
    wherein the controller is configured to manipulate the displayed video image to illustrate the user-specified treatment region;
    wherein the controller further comprises:

        a coordinate converter configured to convert user-specified treatment parameters into selection and modulation commands for a source control and modulation unit;
        the source control and modulation unit, which is configured to drive the radiation source assembly to provide the requisite radiation outputs; and
        an interface management unit including control logic configured to manage the setting of the treatment region and, once the treatment is determined and requested, to control the creation of control sequences to cause treatment through the coordinate converter and the source control and modulation unit;
        wherein the treatment region forms a subset of the field-of-view, and wherein the controller is configured to turn the therapeutic beam on when the scanned beam enters the treatment region, and to turn the therapeutic beam off as the scanned beam exits the treatment region.

2.  The medical device of claim 1, wherein the reflector oscillates at a natural resonant frequency in at least one axis.

3.  The medical device of claim 1 wherein the controller is configured to modulate sensitivity of the receiving system with delivery of the therapeutic beam to inhibit overload.

4.  The medical device of claim 1, wherein the optical fiber is arranged and configured to receive the imaging beam and the therapeutic beam generated by the at least two radiation sources.

5.  The medical device of claim 1 further comprising a motion sensing system including a motion sensor for use in detecting relative movement between the motion sensor and the field-of-view.

6.  The medical device of claim 1, wherein the optical fiber is a single mode fiber.

7. The medical device of claim 1, wherein the radiation source assembly is configured to output an aiming beam to highlight an area of the field-of-view.

**Patentansprüche**

1. Medizinische Einrichtung, umfassend:

eine Strahlungsquellenbaugruppe mit mindestens zwei Strahlungsquellen, wobei eine oder mehrere der Strahlungsquellen dafür ausgelegt sind, einen Bildgebungsstrahl zur Verwendung bei der Visualisierung einer Szene zu erzeugen, und eine oder mehrere der Strahlungsquellen dafür ausgelegt sind, einen therapeutischen Strahl zur Behandlung einer Erkrankung zu erzeugen;
eine optische Faser zum Leiten von Strahlungsenergie von der Strahlungsquellenbaugruppe zu einem distalen Ende der medizinischen Einrichtung in Form eines Strahls;
einen Reflektor, der den Strahl von der optischen Faser empfängt, wobei der Reflektor dafür konfiguriert ist, den Strahl in einem Muster zu scannen, wobei das Ausmaß des durch den gescannten Strahl gebildeten Musters ein Sichtfeld definiert;
ein Empfangssystem mit einem Detektor, der dafür ausgelegt und konfiguriert ist, Strahlung aus dem Sichtfeld zu empfangen, um ein betrachtbares Bild zu erzeugen;
eine Steuerung, die dafür konfiguriert ist, ein Videobild auf der Basis von elektrischen Signalen zu erzeugen, die durch den Detektor erzeugt werden, die der durch den Detektor aus dem Sichtfeld empfangenen Strahlung entsprechen;
eine Geometrieeingabeeinrichtung, die zur Benutzerspezifikation einer Behandlungsregion konfiguriert ist;
eine Anzeigeeinrichtung, die dafür konfiguriert ist, das Videobild des Sichtfelds dem Benutzer anzuzeigen;
wobei der Bildgebungsstrahl und der therapeutische Strahl aus den mindestens zwei Strahlungsquellen so gerichtet werden, dass sie einem gemeinsamen Pfad zu dem Reflektor folgen;
wobei die Steuerung dafür ausgelegt ist, das angezeigte Videobild zu manipulieren, um die vom Benutzer spezifizierte Behandlungsregion darzustellen;
wobei die Steuerung ferner Folgendes umfasst:

einen Koordinatenumsetzer, der dafür ausgelegt ist, vom Benutzer spezifizierte Behandlungsparameter in Auswahl- und Modulationsbefehl für eine Quellensteuer- und -Modulationseinheit umzusetzen;
die Quellensteuer- und -Modulationseinheit, die dafür ausgelegt ist, die Strahlungsquellenbaugruppe so anzusteuern, dass die erforderlichen Strahlungsausgaben bereitgestellt werden; und
eine Schnittstellenverwaltungseinheit mit Steuerlogik, die dafür konfiguriert ist, die Einstellung der Behandlungsregion zu verwalten und, nachdem die Behandlung bestimmt und angefordert wurde, die Erzeugung von Steuersequenzen zu steuern, um Behandlung durch den Koordinatenumsetzer und die Quellensteuer- und -Modulationseinheit zu bewirken;
wobei die Behandlungsregion eine Teilmenge des Sichtfelds bildet und wobei die Steuerung dafür ausgelegt ist, den therapeutischen Strahl einzuschalten, wenn der gescannte Strahl in die Behandlungsregion eintritt, und den therapeutischen Strahl auszuschalten, wenn der gescannte Strahl die Behandlungsregion verlässt.

2. Medizinische Einrichtung nach Anspruch 1, wobei der Reflektor mit einer natürlichen Resonanzfrequenz in mindestens einer Achse oszilliert.

3. Medizinische Einrichtung nach Anspruch 1, wobei die Steuerung dafür ausgelegt ist, die Empfindlichkeit des Empfangssystems bei Verabreichung des therapeutischen Strahls zu modulieren, um Überlastung zu hemmen.

4. Medizinische Einrichtung nach Anspruch 1, wobei die optische Faser dafür ausgelegt und konfiguriert ist, den Bildgebungsstrahl und den therapeutischen Strahl, die durch die mindestens zwei Strahlungsquellen erzeugt werden, zu empfangen.

5. Medizinische Einrichtung nach Anspruch 1, die ferner ein Bewegungserfassungssystem mit einem Bewegungssensor zur Verwendung beim Detektieren von Relativbewegung zwischen dem Bewegungssensor und dem Sichtfeld umfasst.

6. Medizinische Einrichtung nach Anspruch 1, wobei die optische Faser eine Einmodenfaser ist.

**7.** Medizinische Einrichtung nach Anspruch 1, wobei die Strahlungsquellenbaugruppe dafür konfiguriert ist, einen Zielstrahl auszugeben, um einen Bereich des Sichtfelds hervorzuheben.

**Revendications**

**1.** Dispositif médical comprenant :

un ensemble de sources de rayonnement présentant au moins deux sources de rayonnement, une ou plusieurs des sources de rayonnement étant adaptées pour délivrer un faisceau de formation d'image destiné à être utilisé pour visualiser une scène et une ou plusieurs des sources de rayonnement étant adaptées pour délivrer un faisceau thérapeutique destiné à traiter un état médical,
une fibre optique qui envoie sous la forme d'un faisceau l'énergie radiante de l'ensemble de sources de rayonnement vers l'extrémité distale du dispositif médical,
un réflecteur qui reçoit le faisceau de la fibre optique, le réflecteur étant configuré pour balayer le faisceau selon un motif, l'étendue du motif formé par le faisceau balayé définissant le champ de vision,
un système de réception qui comprend un détecteur agencé et configuré pour recevoir le rayonnement du champ de vision en vue de produire une image visible,
un contrôleur configuré pour former une image vidéo sur base des signaux électriques délivrés par le détecteur en correspondance au rayonnement reçu par le détecteur depuis le champ de vision,
un dispositif d'entrée géométrique configuré pour permettre à l'utilisateur de spécifier la région de traitement,
un dispositif d'affichage configuré pour afficher à l'utilisateur l'image du champ de vision,
le faisceau d'imagerie et le faisceau thérapeutique provenant des deux ou plusieurs sources de rayonnement étant orientés de manière à suivre un parcours commun vers le réflecteur,
le contrôleur étant configuré pour manipuler l'image vidéo affichée de manière à illustrer la zone de traitement spécifiée par l'utilisateur,
le contrôleur comprenant en outre :

un convertisseur de coordonnées configuré pour convertir des paramètres de traitement spécifiés par l'utilisateur en commandes de sélection de modulation d'une unité de contrôle et de modulation de la source, l'unité de contrôle et de modulation de la source configurée pour entraîner l'ensemble de sources du rayonnement de manière à ce qu'elles délivrent le rayonnement requis et
une unité de gestion d'interface qui comprend une logique de contrôle configurée pour gérer la définition de la zone de traitement et, une fois que le traitement est terminé et lorsque nécessaire, pour commander la formation de séquences de contrôle qui amènent le traitement par le convertisseur de coordonnées et l'unité de contrôle ou de modulation de la source,
la zone de traitement formant un sous-ensemble du champ de vision, le contrôleur étant configuré pour brancher le faisceau thérapeutique lorsque le faisceau scanné pénètre dans la zone de traitement et pour arrêter le faisceau thérapeutique lorsque le faisceau scanné quitte la zone de traitement.

**2.** Dispositif médical selon la revendication 1, dans lequel le réflecteur oscille à une fréquence naturelle de résonnance suivant au moins un axe.

**3.** Dispositif médical selon la revendication 1, dans lequel le contrôleur est configuré pour moduler la sensibilité du système de réception lorsque le faisceau thérapeutique est envoyé, pour empêcher les surcharges.

**4.** Dispositif médical selon la revendication 1, dans lequel la fibre optique est agencée et configurée de manière à recevoir le faisceau d'imagerie et le faisceau thérapeutique produits par les deux ou plusieurs sources de rayonnement.

**5.** Dispositif médical selon la revendication 1, comprenant en outre un système de détection de déplacement comprenant une sonde de déplacement destinée à être utilisée pour détecter un déplacement relatif entre la sonde de déplacement et le champ de vision.

**6.** Dispositif médical selon la revendication 1, dans lequel la fibre optique est une fibre optique monomode.

**7.** Dispositif médical selon la revendication 1, dans lequel l'ensemble de sources de rayonnement est configuré pour délivrer un faisceau de visée de manière à mettre en évidence une partie du champ de vision.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050020926 A
- US 20060195014 A
- US 5200819 A
- US 873540 A
- US 11615140 B
- US 6140979 A
- US 6245590 B
- US 6285489 B
- US 6331909 B
- US 6362912 B
- US 6384406 B
- US 6433907 B
- US 6512622 B
- US 6515278 B
- US 6515781 B
- US 6525310 B

**Non-patent literature cited in the description**

- **HAMMOND S ; DUNKI-JACOBS R ; HARDY R ; TOPKA T.** Architecture and Operation of a Systolic Sparse Matrix Engine. *Proceedings of the Third SIAM Conference on Parallel Processing for Scientific Computing,* 1987, 419-423